# EUROPEAN PATENT APPLICATION

(11) **EP 3 923 293 A1**
(43) Date of publication of application: **15.12.2021**
(21) Application number: 20178943.5
(22) Date of filing: 09.06.2020
(51) Int. Cl.: G16H 30/00

(54) **SYSTEM AND METHOD FOR ANALYSIS OF MEDICAL IMAGE DATA BASED ON AN INTERACTION OF QUALITY METRICS**

(71) Applicant: Koninklijke Philips N.V., 5656 AG Eindhoven (NL)
(72) Inventor: GOOßEN, André, 5656 AE Eindhoven (NL); BRUECK, Matthias, 5656 AE Eindhoven (NL); von Berg, Jens, 5656 AE Eindhoven (NL); KROENKE, Sven, 5656 AE Eindhoven (NL); BYSTROV, Daniel, 5656 AE Eindhoven (NL); YOUNG, Stewart, 5656 AE Eindhoven (NL)
(74) Representative: Philips Intellectual Property & Standards

(57) **Abstract**

The disclosure relates to a system for analysis of medical image data, which represents a two-dimensional or three-dimensional medical image. The system is configured to read and/or determine, for the medical image, a plurality of image quality metrics and to determine a combined quality metrics based on the image quality metrics. The system is further configured so that the determination of the combined quality metrics takes into account an interaction between the image quality metrics in their combined effect on the combined quality metrics.

## Description

### FIELD OF THE INVENTION

The invention relates to a system, method, a program element and a computer readable medium and for analysis of medical image data. Specifically, the analysis is performed so that it takes into account an interaction between a plurality of image quality metrics in their combined effect on a combined quality metrics.

### BACKGROUND OF THE INVENTION

In radiology departments, the functions of generating a radiograph and assessing its diagnostic quality are divided between the radiographer, also denoted as radiologic technologist, who operates the image acquisition unit on the one hand and the radiologist on the other hand. If an image shows severe quality degradation, for example caused by errors in positioning the patient relative to the imaging unit, the radiologist, when performing reject analysis, rejects the image classifying it as not being diagnostic. In such cases, it is necessary to acquire a repeat image from the patient. Repeat images, however, lead to additional costs, less productivity in the radiology department and higher radiation doses given to the patient. They also do not fit into a clinical workflow, which has to manage an ever-increasing amount of diagnostic data.

A similar problem exists in that some radiographers tend to directly reject images without consulting the radiologist in an effort to anticipate a possible rejection by the radiologist. This, however, leads to unnecessary repeat images, since the image was rejected by somebody who only has a basic medical training and is therefore not qualified to make a determination on whether or not the image is suitable for diagnosis.
Due to the deficiencies in current processes for generating medical images and assessing their diagnostic quality, it would be desirable to have a more efficient system for analyzing medical image data.

### SUMMARY OF THE INVENTION

The present disclosure pertains to a system for analysis of medical image data, which represent a two-dimensional or three-dimensional medical image, which has been acquired from a human subject. The system comprises a data processing system, which is configured to read and/or determine, for the medical image, a plurality of image quality metrics. The data processing system is further configured to determine a combined quality metrics based on the image quality metrics. The data processing system is configured so that the determination of the combined quality metrics takes into account an interaction between the image quality metrics in their combined effect on the combined quality metrics.

The image data may be acquired using a medical imaging unit. The medical imaging unit may include an X-ray imaging unit and/or a magnetic resonance tomography unit and/or an ultrasonic imaging unit. The X-ray imaging unit may be configured for plane radiography and/or for X-ray computed tomography.

The acquired medical images of the human subjects may include images of a lung, a chest, a rib, bones, an ankle joint or a knee joint.

The three-dimensional medical images may represent tomographic images. The tomographic images may be generated from two-dimensional images using a reconstruction algorithm. The two-dimensional medical images may be generated using projection imaging, in particular using projectional radiography.

The data processing system may be configured to read at least a portion of the quality metrics from one or more files, which are stored in a storage device of the data processing system. Additionally or alternatively, the data processing system may be configured to determine at least a portion of the quality metrics using an analysis algorithm, which uses at least a portion of the medical image data as input. The analysis algorithm may include, but is not limited to, one or a combination of a segmentation algorithm, a registration algorithm for registering the image with an atlas, a classifier, a machine-learning algorithm. The machine-learning algorithm may be configured for supervised and/or unsupervised learning. Specifically, the machine-learning algorithm may be an artificial neural network (ANN).

Additionally or alternatively, the data processing system may be configured to receive at least a portion of the quality metrics via a user interface of the data storage system and/or from a further data processing system. The data processing system may determine the image quality metrics automatically or interactively (i.e. requiring user intervention).

One or more or each of the quality metrics may relate to a parameter of an imaging process for acquiring the medical image using the medical imaging unit. One or more or each of the quality metrics may be indicative of or may include a parameter of a position and/or an orientation of a body portion of the human subject relative to a component of the medical imaging unit. By way of example, the components may include a detector of the imaging unit, such as an X-ray detector and/or a radiation source of the imaging unit. One example of an orientation parameter is the rotation angle of an ankle about a longitudinal axis. One example of a position parameter is the position of the lung field relative to the field of view of an X-ray radiation sensitive detector. The field of view may be adjusted by adjusting a position of the collimator of the X-ray imaging unit and/or by adjusting a relative position between the subject and the radiation-sensitive detector. Additionally or alternatively, the extent of the lung field relative to the field of view can be adjusted by adjusting an opening formed by the collimator.

Additionally or alternatively, one or more or each of the quality metrics may be indicative of, or may include a parameter of an orientation of portions of the human subject relative to each other. For one or more or all of the body portions, the respective body portion may be imaged in the medical image. One example of an orientation of two body portions relative to each other is the flexion angle of an ankle (i.e. angle of dorsiflexion or plantarflexion).

The combined quality metric may be indicative of whether or not, or to which degree, the medical image is sufficient for a medical diagnosis. The data processing system may be configured to determine the combined quality metric using an algorithm stored in the data processing system. The determination of the combined quality metric may be performed automatically or interactively (i.e. requiring human intervention). By way of example, the data processing system may receive, via a user interface of the data processing system, user input, which is used for determining the combined quality metric. One example for such a user input is the intended diagnosis based on the medical image. Based on this user input, the data processing system may select one or more image quality metrics and/or an algorithm for determining the combined quality metric. Thereby, it is possible to determine a combined quality metric, which reliably allows determination whether the acquired image is suitable for making the intended diagnosis.

At least a portion of the combined quality metric may be a value of a plurality of predefined quality levels. One or more of the quality levels may indicate that the medical image meets a predefined criterion and one or more second levels may indicate that the medical image does not meet a predefined criterion. The predefined criterion may be indicative of whether or not, or to which degree the medical image is suitable for a predefined diagnosis.

The combined quality metric may be a tabulated function or an analytical function, which depends on the image quality metrics. The combined quality metric may include or may be a scalar value, a vector and/or a state variable. Combinations of values of the image quality metrics, which represent a same value of the combined quality metrics may represent a contour line in a height map, which is indicative of at least a portion of the combined quality metric.

According to a further embodiment, the interaction between the image quality metrics is so that a change of the combined quality metrics, which is caused by a change of a first one of the quality metrics is compensable by a change of one or more of the remaining quality metrics.

According to a further embodiment each of the image quality metrics is associated with a respective predefined optimum value or predefined optimum range. By way of example, one of the optimum values may be an optimum orientation of a body portion, in particular an imaged body portion relative to a component of the imaging unit, such as a detector and/or a source of the medical imaging unit. Additionally or alternatively, the optimum value may be an optimum orientation of body portions relative to each other. By way of example, the optimum angle may be an optimum angle of the ankle (i.e. angle of dorsiflexion or angle of plantarflexion).

The interaction between the image quality metrics may be so that an increased deviation from the optimum range or optimum value of a first one of the metrics may be compensable by a decreased deviation from the optimum range or optimum value of one or more of the remaining image quality metrics.

According to a further embodiment, the one or more or each of the quality metrics is indicative of one or more parameters of a position and/or one or more parameters of an orientation of an imaged body portion relative to a component of the imaging unit or relative to a further body portion. The component may be a detector and/or a source of the medical imaging unit. One or more or each of the body portions may be at least partially imaged in the medical image.

Further examples of quality metrics are but are not limited to: and inhalation status, a collimation parameter a field of view position parameter and a distance between the body portion and the component of the medical imaging unit.

By way of example, the inhalation state may be expressed using a number of ribs, which are shown in the image above the diaphragm. The analysis algorithm may be configured to detect the ribs, which are shown in the medical image above the diaphragm and determine the number of these ribs. Detection of the ribs may be performed using a segmentation algorithm.

Determination of the collimation parameter may include determining a distance between a border of a field of view of the image and an optimum collimation border that covers the target anatomy (such as the lung field) without under-collimation or over-collimation. The collimation parameter may be configured to allow detection of under-collimation and over-collimation in the medical images. Over-collimated images show only a portion of the target anatomy (such as the lung field) so that only a portion of the necessary information is available to perform a diagnosis. On the other hand, under-collimated images show body portions which do not need to be imaged to perform the diagnosis so that these body portions are exposed to radiation, which can be avoided through proper collimation.

The field of view position parameter may be a parameter of a position of the field of view of the image relative to an optimum field of view, which covers the target anatomy without under-collimation or over-collimation.

Other quality metrics may include but are not limited to: (a) a metric, which is indicative of whether or not predetermined anatomical structures, or a minimum amount of anatomical structures are visible in the image (e.g. whether one or both scapula are visible in the imaged lung field), and (b) a metric, which is indicative of a percentage of overlap between congruent anatomical structures (e.g. femoral condyles). Congruent anatomical structures may be defined as structures that are either symmetric or nearly identical. It may be advantageous , in particular for a two-dimensional projection image, to bring the congruent anatomical structures into overlap or substantial overlap with respect to their contours. This may increase the reliability of the diagnosis.

According to a further embodiment, the system is configured to determine, for one or more of the images, based on the determined combined quality metric, a parameter or state variable representing a change of imaging conditions for using the imaging unit. By way of example, the state variable represents an operation state of the medical imaging unit. Further, examples for the parameter representing the change of imaging conditions are but are not limited to: (a) an operation parameter of the medical imaging unit, (b) a parameter of a position and/or orientation of a body portion relative to a component of the medical imaging unit, and (c) a parameter of a position and/or orientation of two or more body portions relative to each other.

By way of example, the change in imaging conditions may be a change in a position and/or orientation of the body portion relative to the component of the imaging unit and/or a change in a position and/or orientation of body portions relative to each other. Additionally or alternatively, the change in imaging conditions is a change in operational parameters of the imaging unit, such as a position of a collimator, a tilt of the X-ray tube and/or X-ray sensitive detector, the position of an anti-scatter grid, or whether or not to use an anti-scatter grid, a geometrical configuration of a patient support device, such as the geometrical configuration of a flexion unit.

The changed imaging conditions may cause a change of one or more of the image quality metrics. In other words, the system may provide a recommendation for the user or for the medical imaging unit to adapt the imaging conditions so as to obtain a change in the combined quality metric.

According to a further embodiment, the determination of the image quality metrics includes segmenting at least a portion of the medical image. Additionally or alternatively, the determination comprises registering the medical image with an atlas. The registration of the image data with the atlas may be performed using the segmented image. The image segmentation may be configured to determine one or more image regions. The segmentation may be performed using the data processing system and/or using a further data analysis system from which the data processing system receives data.

The segmentation of the medical image may be performed using one or a combination of the following segmentation techniques: thresholding, region growing, Watershed transform, edge detection, shape model, appearance model and hand-segmentation using user interaction with the graphical user interface. Additionally or alternatively, the segmentation may be performed using an artificial neural network. The segmentation may be performed automatically or interactively (i.e. requiring user intervention). In interactive segmentations, the computer system may receive user input, which is indicative of one or more parameters of a location, an orientation and/or an outer contour of an image region. The artificial neural network may be trained using segmented images, in particular performed by interactive segmentation.

The atlas may include a statistically averaged anatomical map of one or more body portions. At least a portion of the atlas may be indicative of or may represent a two-dimensional or three-dimensional shape of a portion of the body, in particular an anatomical portion of the body. By way of example, at least a portion of the atlas may be indicative of or may represent a three-dimensional outer surface of one or more anatomical or functional portions of the body. By way of example, the anatomical portion of the body may be one or more bones and/or one or more portions of bones.

The atlas may be generated based on anatomical data acquired from a plurality of human bodies. The anatomical data may be acquired using medical imaging units, such as X-ray imaging units, magnetic resonance tomography units and/or ultrasonic imaging units. The human bodies, which are used for generating the atlas data may share a common feature or range of features, such as gender, age, ethnicity, body size, body mass and/or pathologic state.

The present disclosure pertains to a system for analysis of medical image data, which represent a plurality medical images, each of which being a two-dimensional or a three-dimensional image. The medical images are acquired from one or more human subjects using one or more medical imaging units, wherein each of the medical imaging units is configured to acquire medical images. The system comprises a data processing system, which is configured to read and/or generate, for each of the medical images, one or more quality metrics. The data processing system is further configured to receive, via the user interface, for at least a portion of the images, user input indicative of a user-specified quality rating for the respective image. The data processing system is further configured to: (a) determine or adapt an algorithm for determining a combined quality metric based on the image quality metrics and based on the user input; and/or to (b) determine or adapt an algorithm for classifying the images or selecting a portion of the images, wherein the classifying or the selecting is based on the user input and based on the combined quality metric. The combined quality metric depends on an interaction between the image quality metrics in their combined effect on the combined quality metrics.

The data processing system may be configured to read at least a portion of the quality metrics from one or more files, which are stored in a storage device of the data processing system. Additionally or alternatively, the data processing system may be configured to determine at least a portion of the quality metrics using an analysis algorithm, which uses at least a portion of the medical image data as input. The analysis algorithm may include, but is not limited to, one or a combination of a segmentation algorithm, a registration algorithm for registering the image with an atlas, a classifier, a machine-learning algorithm. The machine-learning algorithm may be configured for supervised and/or unsupervised learning. Specifically, the machine-learning algorithm may be an artificial neural network (ANN).

Additionally or alternatively, the data processing system may be configured to receive at least a portion of the quality metrics via a user interface of the data storage system and/or from a further data processing system. The data processing system may determine the image quality metrics automatically or interactively (i.e. requiring user intervention).

The user input, which is indicative of a user-specified quality rating may include one of a plurality of predefined levels. The user input may be received via a user interface. The user interface may include but is not limited to one or a combination of: a keyboard, a computer mouse, a touch control, a voice control and/or gesture control.

The algorithm for classifying the images may be an algorithm for classifying, each of the images, into one or more of a plurality of predefined classes. The classification may include binary classification data and/or probabilistic classification data. Probabilistic classification data may be defined as data, which include one or more probability values for one or more of the predefined classes. Binary classification data may be defined as data, which include for one or more of the predefined classes, either a value which indicates that the image is a member of the class or a value that the image is not a member of the class.

The predefined classes may include a class of diagnostic images and/or a class of non-diagnostic images. The predefined classes may be represented by the class of diagnostic images and the class of non-diagnostic images.

The algorithm for selecting may, for example, an algorithm for selecting a portion of the diagnostic images. In an alternative embodiment, the algorithm for selecting is an algorithm for selecting the non-diagnostic images.

According to a further embodiment, the determining of the algorithm for determining the combined quality metric and/or the determining of the algorithm for classifying the images includes training a machine learning algorithm, in particular an artificial neural network (ANN). The artificial neural network may include an input layer, one or more intermediate layers and an output layer. The artificial neural network may be configured as a convolutional neural network, in particular as a deep convolutional neural network.

According to a further embodiment, the system is configured to display, via the user interface, output, which is indicative of the combined quality metric for at least a portion of the images. Additionally or alternatively, the system may be configured to display output, which is indicative of a classification of the medical images, which classifies the medical images based on the combined quality metric. The output may be indicative of probabilistic classification data and/or binary classification data. The classification may be determined and/or adapted by the data processing system based on the user input, which is indicative of the user-specified quality rating.

According to an embodiment, the data processing system is configured to receive, via the user interface, a user-specified selection of one or more of the medical images for inputting the user input which is indicative of the user-specified quality rating for the images, which are selected by the user-specified selection. The user-specified selection may be received via a graphical user interface, in particular via a computer mouse of the graphical user interface. The data processing system may be configured to request the user, in response to the selection, to input the user input, which is indicative of the user-specified quality rating for the selected one or more images.

According to an embodiment, the system is configured to output, for the at least the portion of the images, output, which is indicative of one or more image quality metrics of the respective image.

According to a further embodiment, the system is configured to output a graphical representation which is indicative of a coordinate system of one or more of the image quality metrics. The graphical representation may be displayed using a graphical user interface. The graphical representation may be displayed on a display device of the data processing system. For one or more of the images, the graphical representation may further be indicative of a spatial relationship between the respective image and the coordinate system. The graphical representation may include, for each of the images, a graphical representation representing an image, such as one or more icons, wherein a position and/or orientation of the graphical representation representing the image, relative to the coordinate system is indicative of the image quality metrics of the respective image.

For each of the images and for the image quality metrics, which are used to form the coordinate system, the spatial relationship may be indicative of the values of the image quality metrics of the respective image. The spatial relationship may be a one-, two- or three-dimensional spatial relationship.

According to the embodiment, the data processing system is configured to receive, via the user interface, a user-specified selection of one or more of the images for which the graphical representation is indicative of the spatial relationship. The user interface may be configured as a graphical user interface in which one or more regions are user-selectable. The selectable regions may be displayed on a display device of the data processing system so that the location and extent of the regions are visually perceptible for the user. A spatial arrangement of the regions may depend on the graphical representation, in particular on the spatial relationship of which the graphical representation is representative. The graphical representation may be indicative of the user-selectable regions.

Each of the user-selectable regions may be user-selectable using a computer mouse of the user interface. Each of the user-selectable regions may represent a medical image. Each of the user-selectable regions may be arranged in a coordinate system of one or more of the image quality metrics. The graphical user interface may display, for each of the user-selectable region, a graphical representation, such as an icon, representing a medical image, which is selectable by the user input.

By way of example, the graphical representation includes a graph. The graph may illustrate the combined quality metric of the images as discrete values of a height function.

The coordinate system may be one, two or three-dimensional. Additionally or alternatively, the coordinate system may be a rectangular, spherical or cylindrical coordinate system. The data processing system may be configured to receive user input indicative of one or more selected image quality metrics. The data processing system may be configured to display a coordinate system, which includes the selected image quality metrics.

According to a further embodiment, the determining or adapting of the algorithm for classifying the images and/or for determining the combined quality metric is performed using one or a combination of: a maximum likelihood model and a machine learning algorithm. The machine-learning algorithm may be configured for supervised or unsupervised learning. The machine learning algorithm may be implemented using a support vector machine or an artificial neural network.
According to an embodiment, the system includes one or more medical imaging units, wherein each of the medical imaging units is configured to acquire medical images.

The present disclosure further pertains to a computer-implemented method for analysis of medical image data, which represent a two-dimensional or three-dimensional medical image, wherein the image is an image of at least a portion of a human subject. The method is performed using a data processing system, wherein the method comprises reading and/or determining, using the data processing system, for the medical image, a plurality of image quality metrics. The method further comprises automatically or interactively determining, using the data processing system, a combined quality metrics based on the image quality metrics. The determining of the combined quality metrics takes into account an interaction between the image quality metrics in their combined effect on the combined quality metrics.

The present disclosure pertains to a method for acquiring and analyzing medical images. The method includes acquiring, using a medical imaging unit a medical image, wherein the method further includes the computer-implemented method described in the previous paragraph for analyzing the acquired medical image.

The present disclosure further pertains to a computer implemented method for analysis of medical image data, which represent a plurality medical images, each of which being a two-dimensional or a three-dimensional image; wherein the medical images are images of portions of one or more human subjects. The method is performed using a data processing system, wherein the method comprises reading and/or generating, using the data processing system, for each of the medical images, one or more quality metrics. The method further includes receiving, via a user interface of the data processing system, for at least a portion of the images, user input indicative of a user-specified quality rating for the respective image. The method further comprises at least one of the following: (a) determining or adapting, using the data processing system, an algorithm for determining a combined quality metric based on the image quality metrics and based on the user input; and/or (b) determining or adapting, using the data processing system, an algorithm for classifying the images or selecting a portion of the images, wherein the classifying or the selecting is based on the user input and based on the combined quality metric. The combined quality metric depends on an interaction between the image quality metrics in their combined effect on the combined quality metrics.

The present disclosure further pertains to a method for acquiring and analyzing medical images. The method includes acquiring, using one or more medical imaging units a plurality of medical images; wherein each of the medical imaging units is configured to acquire medical images; wherein the method further includes the computer-implemented method described in the previous paragraph for analyzing the plurality of medical images.

The present disclosure pertains to a program element for analysis of medical image data, which represent a two-dimensional or three-dimensional medical image, wherein the image is an image of at least a portion of a human subject; wherein the method is performed using a data processing system. The program element, when being executed by a processor of the data processing system, is adapted to carry out reading and/or determining, using the data processing system, for the medical image, a plurality of image quality metrics. The program element, when being executed by a processor, is further configured to carry out automatically or interactively determining, using the data processing system, a combined quality metrics based on the image quality metrics. The determining of the combined quality metrics takes into account an interaction between the image quality metrics in their combined effect on the combined quality metrics.

The present disclosure pertains to a program element for analysis of medical image data, which represent a plurality medical images, each of which being a two-dimensional or a three-dimensional image. The medical images are images of portions of one or more human subjects. The program element, when being executed by a processor of the data processing system, is adapted to carry out reading and/or generating, using the data processing system, for each of the medical images, one or more quality metrics. The program element is further adapted to carry out receiving, via a user interface of the data processing system, for at least a portion of the images, user input indicative of a user-specified quality rating for the respective image. The program element, when being executed by a processor, is further adapted to carry out at least one of the following (a) determining or adapting, using the data processing system, an algorithm for determining a combined quality metric based on the image quality metrics and based on the user input; and/or (b) determining or adapting, using the data processing system, an algorithm for classifying the images or selecting a portion of the images, wherein the classifying or the selecting is based on the user input and based on the combined quality metric. The combined quality metric depends on an interaction between the image quality metrics in their combined effect on the combined quality metrics.

The present disclosure pertains to a computer program product having stored thereon the computer program element of any one of the above-described embodiments.

### BRIEF DESCRIPTION OF THE DRAWINGS

Fig. 1 is a schematic illustration of a system for analysis of medical image data according to an exemplary embodiment;
Fig. 2 schematically displays two image quality metrics of an ankle radiograph, which are determined by the system according to the exemplary embodiment;
Fig. 3 schematically illustrates an image display window of a graphical user interface of the system according to the exemplary embodiment, which displays a normalized X-ray radiograph;
Fig. 4 schematically illustrates the dependency of a combined quality metric on two image quality metrics, wherein the combined quality metrics is calculated by computer systems of the system according to the exemplary embodiment;
Fig. 5 schematically illustrates a graphical representation of a user interface of a central computer system of the system according to the exemplary embodiment, wherein for each of the images, the graphical representation is indicative of the value of the combined quality metric as well as the image quality metrics of the respective image;
Fig. 6 schematically illustrates the operation of the graphical user interface of the central computer system; and
Fig. 7 is a schematic illustration of an artificial neural network (ANN) used by computer systems of the system according to the exemplary embodiment.

### DETAILED DESCRIPTION OF EMBODIMENTS

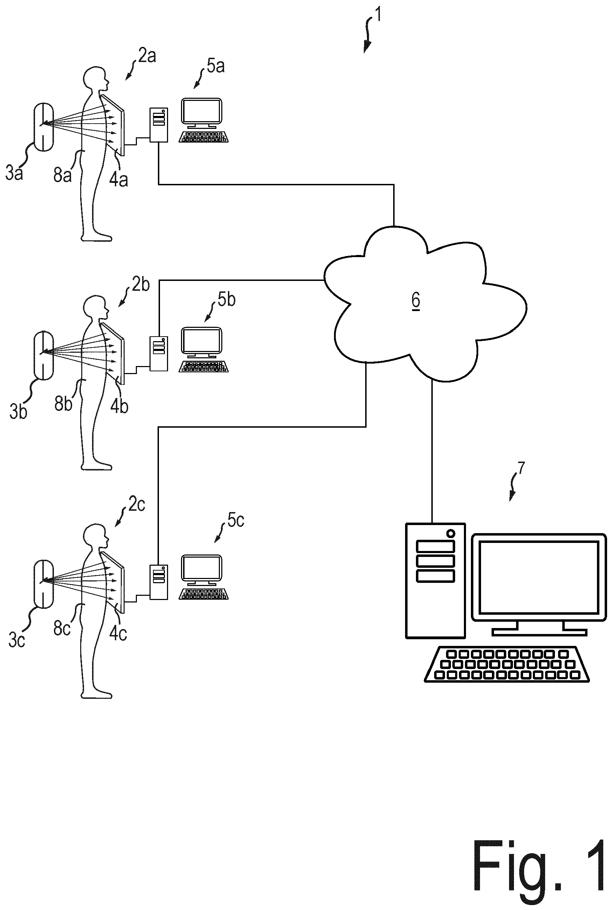

Fig. 1 is a schematic illustration of a system 1 for analysis of medical image data according to an exemplary embodiment. The system 1 includes a plurality of medical imaging units 2a, 2b, 2c, each of which being configured to acquire X-ray images using planar projection radiography. Each of the medical imaging units 2a, 2b, and 2c include a radiation source 3a, 3b, 3c and a radiation-sensitive detector 4a, 4b, 4c, which is configured to detect X-ray imaging radiation emitted from the radiation source 3a, 3b, 3c.

Each of the imaging units 2a, 2b, 2c is configured to acquire projection X-ray images from a subject 8a, 8b, 8c, which is positioned between the radiation source 3a, 3b, 3c and the radiation-sensitive detector 4a, 4b, 4c. The system 1 includes, for each of the medical imaging units 2a, 2b, 2c, a computer system 5a, 5b, 5c, which is operated by a radiographer who positions the subject 8a, 8b, 8c between the X-ray-radiation source 3a, 3b, 3c and the radiation-sensitive detector 4a, 4b, 4c so that a body portion of the subject 8a, 8b, 8c is imaged using the X-rays emitted from the radiation source 3a, 3b, 3c.

As is illustrated in Fig. 1, the imaged body portion of the subject 8a, 8b, 8c may be a chest of the subject 8a, 8b, 8c so that the image data, which are generated using the medical imaging units 2a, 2b, 2c represent chest radiographs. However, it is also conceivable that the acquired image data represent other body portions, such as ankles, knees, shoulders elbows, wrists, hips or spine.

The system 1 further comprises a central computer system 7, which may be, for example, operated by a radiologist. The central computer system 7 may be a dedicated server that receives all images acquired using the medical imaging units 2a, 2b and 2c for analysis by the radiologist. The computer systems are in signal communication with each other via a computer network, which may include a LAN 6 (local area network) and/or the Internet.

Each of the medical imaging units 2a, 2b and 2c is configured to determine, for each of the acquired images, a plurality of image quality metrics based on the image data of the respective image. The determination of the imaging quality metrics may be performed automatically or semi-automatically (i.e. requiring user intervention). One or more of all of the image quality metrics may be indicative of, or may be a parameter of, a position and/or an orientation of the imaged body portion of the imaged subject 8a, 8b, 8c relative to a component of the medical imaging unit 2a, 2b, 2c. The component may include at least a portion of the X-ray radiation source 3a, 3b, 3c and/or the radiation-sensitive detector 4a, 4b, 4c.

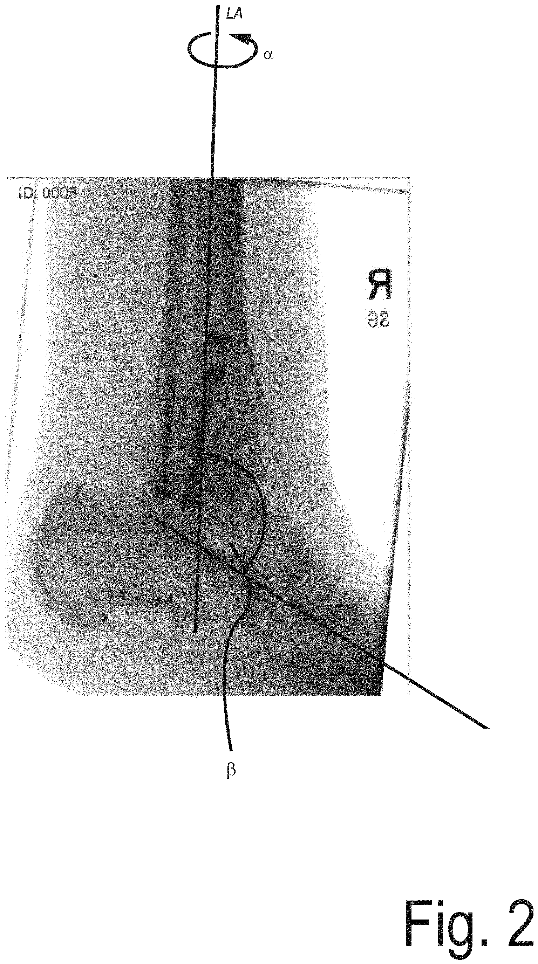

Fig. 2 is a schematic illustration of an X-ray radiograph of an ankle, which is acquired by one of the imaging units 2a, 2b and 2c (shown in Fig. 1). In some X-ray examinations of ankles, it is advantageous if the rotation angle about a longitudinal axis of the tibia (designated in Fig. 2 as α) as well as a flexion angle of the ankle (i.e. angle of dorsiflexion and plantarflexion, which is designated in Fig. 2 as β) are within predefined ranges so that the radiograph exhibits diagnostic features, such as joint spaces, which allow the radiologist to make a medical diagnosis based on the radiograph. Further, radiographs, for which these parameters are not within the predefined ranges can show artifacts, which can make it difficult or even impossible to make a reliable diagnosis. Therefore, these parameters represent image quality metrics, which may be used to determine whether or not the radiograph has a diagnostic image quality.

It is to be noted that these image quality metrics are only examples and the present invention is not limited to these image quality metrics. Specifically, the image quality metrics, as well as the predefined ranges and/or the optimum values for the image quality metrics, may depend on the imaged body portion and/or on a certain diagnosis which is intended to be made based on the radiograph.

The determination of one or more or all of the image quality metrics may be performed using image processing applied to the image. The image processing may include a segmentation of the image. The segmentation of the medical image may be performed using one or a combination of the following segmentation techniques: thresholding, region growing, Watershed transformation, edge detection, using a shape model, using an appearance model and hand-segmentation using user interaction with the graphical user interface. Additionally or alternatively, the segmentation may be performed using an artificial neural network. The artificial neural network may be trained using hand segmentation. The segmentation may be performed automatically or interactively (i.e. requiring user intervention). In interactive segmentations, the computer system may receive user input, which is indicative of one or more parameters of a location, an orientation and/or an outer contour of an image region.

The segmentation may result in one or more segmented image regions and/or in one or more contours. The image regions and/or contours may be two-dimensional. In three-dimensional images, the image regions or contours may be three-dimensional. At least a portion of the image regions or contours may represent an anatomical or functional portion of the body or a surface thereof. An anatomical portion of the body may be a bone structure and/or a tissue structure of the body. A functional portion of the body may be a portion of the body which performs an anatomical function.

Additionally or alternatively, the determination of the image quality metrics may include registering at least a portion of the image with an atlas. The data processing system may be configured to extract features from the image, which are used for registering the image with the atlas. The features may be extracted using image processing. The atlas registration may be performed based on the segmentation of the image. Additionally or alternatively, the atlas registration may be performed based on landmarks, which are detected in the image using the computer system.

The segmentation of the image may be configured to extract one or more parameters of a position, one or more parameters of an orientation and/or one or more parameters of an extent and/or a shape of a portion of the body, such as an anatomical or functional portion of the body. The atlas registration may use one or more of the extracted parameters to register the image with the atlas.

It has been shown by the inventors that the atlas registration leads to a more reliable determination of the image quality metrics. Thereby, the combined quality metrics, which is determined based on the determined image quality metrics and which will be explained in more detail below, may more accurately indicate, whether or not an image is of diagnostic image quality. However, it is also been shown that a sufficient accuracy can be obtained without using segmentation and atlas registration techniques.

Each of the computer systems 5a, 5b and 5c (shown in Fig. 1) is configured to display to the user, via a graphical user interface, the image. The computer systems may further display, concurrently with the image, a graphical representation, which is at least partially indicative of one or more of the features, which are used to register the image with an atlas.

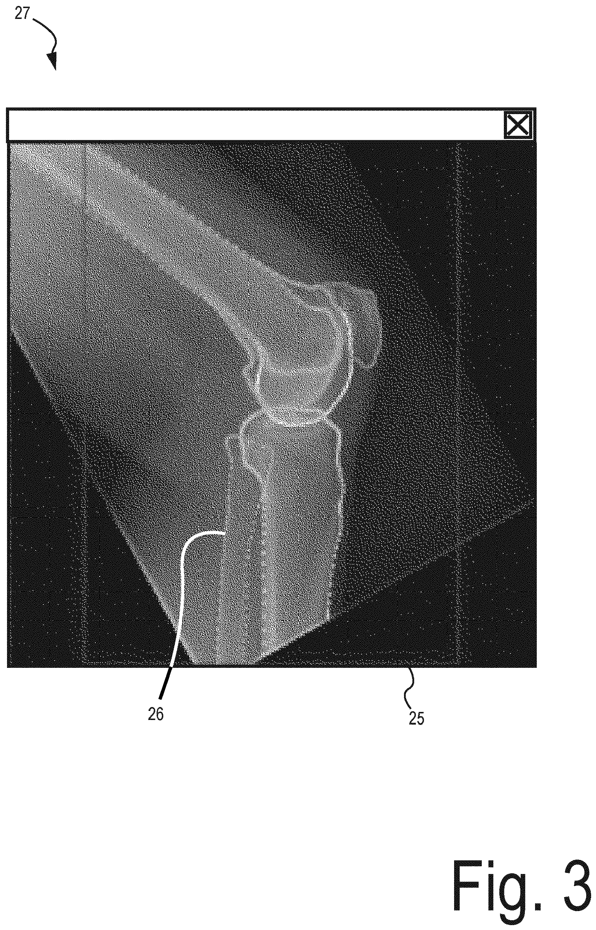

Fig. 3 is a schematic illustration of an image display window 27 of the graphical user interface showing an X-ray image 25 of a knee joint and a graphical representation 26, which is indicative of the outer contour of the bones (i.e. the tibia, the fibula, the patella and the femur). In the illustrated exemplary embodiment, the computer system uses at least a portion of these outer contours to register the image with the atlas. The graphical representation, which is indicative of parameters, which have been used for registering the image with the atlas (as it is, for example, illustrated in Fig. 3) allows the user to verify, whether the computer system has correctly determined the image quality parameters.

Each of the computer systems 5a, 5b and 5c is configured to determine, for each of the medical images, at least one combined quality metric based on the image quality metrics of the respective image. By way of example, the combined quality metric includes a parameter, a vector and/or a state variable which are calculated based on the calculated values of the image quality metrics. The combined quality metric may be a function of the image quality metrics. The function may include an analytical function and/or a tabulated function, which are stored in the computer systems 5a, 5b, 5c.

The inventors have found that the image quality metrics interact in their effect on the image quality, which is a measure of the suitability of the medical image for medical diagnosis.

By way of example, for ankle radiographs (such as the radiograph shown in Fig. 2), the inventors have found that a larger deviation of the rotation angle α about the tibia's longitudinal axis *LA* can be compensated by a smaller deviation of the flexion angle β of the ankle joint. In other words, for images in which the deviation of the flexion angle β from an optimum flexion angle is comparatively small, the rotation angle α about the tibia's longitudinal axis can have a comparatively large deviation from the optimum rotation angle.

Additionally or alternatively, for images in which the deviation of the rotation angle α about the tibia's longitudinal axis from the optimum rotation angle is comparatively small, the flexion angle β of the ankle joint can have a comparatively large deviation from the optimum flexion angle.

The inventors have further shown that a combined quality metric, which takes into account this interaction between image quality metrics allow a more accurate determination of whether or not an image is of diagnostic quality.

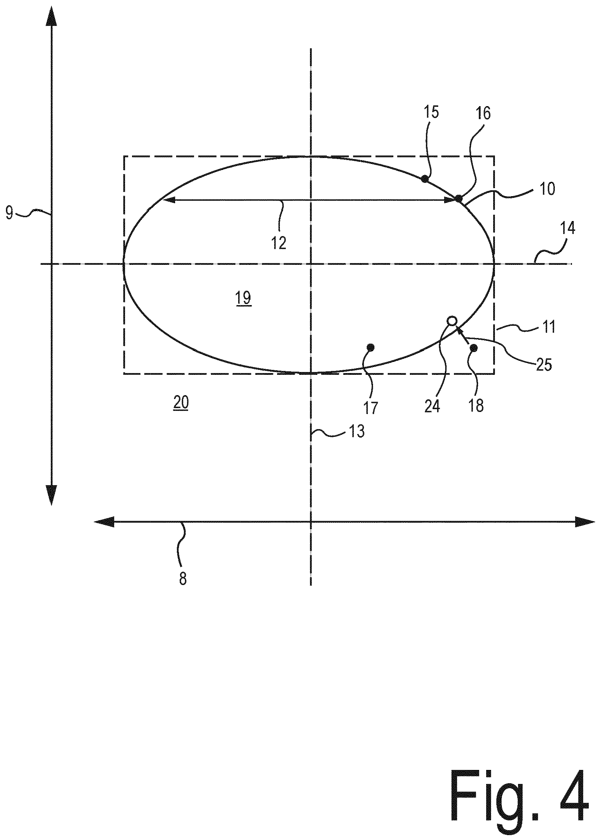

Characteristics of such a combined quality metrics, which takes into account the interaction between the image quality metrics, are schematically illustrated in Fig. 4. The diagram of Fig. 4 shows a contour line 10 of equal height, which represents combinations of a first image quality metric (represented by the x-axis 8) and a second image quality metric (represented by the y-axis 8), for which the combined quality metrics has a constant value. In the exemplary embodiment of Fig. 4, the first image quality metric (represented by the x-axis 8) is the rotation angle about the tibia's longitudinal axis (designated in Fig. 2 as α) and the second image quality metric (represented by the y-axis 9) is the flexion angle of the ankle joint (designated in Fig. 2 as β). It is to be noted, however, that the invention is not limited to this combination of image quality metrics. Each of the image quality metrics has an optimum value, which are illustrated in Fig. 4 by lines 13 and 14.

Since the computer systems 5a, 5b and 5c (shown in Fig. 1) determine, for each of the acquired images the flexion angle β and the rotation angle α about the tibia's longitudinal axis, in the diagram of Fig. 4, each image represents a point (such as the points 15, 16, 17 and 18). The value of the combined quality metrics, which is represented by contour line 10 is selected to represent a limit, which separates a first region 19 from a second region 20. The first region 19 represents images, for which the first and second image quality metrics are so that the image is suitable for diagnosis. The second region 20 represents images for which the first and second image quality metrics are so that the image is unsuitable for diagnosis.

Therefore, the limit, which is represented by the contour line 10 represents a criterion for selecting images based on the combined quality metric.

Since the images represented by points 15, 16 and 17 are within the region limited by the contour line 10 or at the contour line 10, these images are of diagnostic image quality. On the other hand, image 18 is outside the contour line 10 so that this image is of a non-diagnostic image quality.

Since the contour line 10 for the combined quality parameter deviates from the shape of a rectangle 11, the range 12 within which the rotation angle about the tibia's longitudinal axis (parameter α in Fig. 2) is acceptable depends on the value of the flexion angle of the ankle joint (parameter β in Fig. 2). Specifically, as can be seen by comparing, images 15 and 16 shown in Fig. 4, a smaller deviation of the flexion angle (such as in image 16) from its optimum value 14 allows for a greater deviation of the rotation angle about the tibia's longitudinal axis from its optimum value 13. As can further be seen from Fig. 4, this situation is different from the situation (represented by rectangle 11) in which fixed limits are defined for each of the image quality metrics.

Each of the computer systems 5a, 5b and 5c (shown in Fig. 1) is configured to output the combined quality metric, or an output, which is determined based on the combined quality metric, to the radiographer via the user interface. This allows the radiographer to see, whether the image is suitable for diagnosis so that a repeat image can be acquired, if necessary. Since the determination of the combined quality metric is performed automatically based on the calculated image quality and metrics, it is not necessary for the radiologist to be present, when the radiograph is acquired.

The output may further be configured so that it is indicative for a change in imaging conditions so that the resulting image is suitable (or better suitable) for diagnosis.

By way of example, for the image 18, which is shown in Fig. 4 there is a comparatively large deviation from the optimum rotation angle 14 and a comparatively small deviation from the optimum flexion angle 13. However, since the combined quality metric considers the interaction between the image quality metrics, only small corrections of both angles of an approximately equal amount are necessary (as indicated by arrow 25) for obtaining an image 24, which is suitable for diagnosis.

The determined image quality metrics and the combined quality metrics are transmitted from the computer systems 5a, 5b and 5c (shown in Fig. 1) to the central computer system 7 for further analysis performed by the radiologist.

The computer system 7 includes a user interface allowing the radiologist to review the determined combined quality metrics of a plurality of images acquired using the imaging units 2a, 2b and 2c. Based on the review, the central computer system 7 and/or the computer systems 5a, 5b and 5c are able to adjust the algorithm for determining the combined quality metrics or to adjust the algorithm for determining, based on the combined quality metrics, whether or not the image is a diagnostic image.

Therefore, the central computer system 7 is configured to determine, based on a user-specified quality rating for one or more of the images (provided by the radiologist), data, which is indicative of a criterion for selecting images (such as diagnostic images) based on the combined quality metric. Further, the central computer system 7 is configured to determine, based on the user specified quality rating, data, which are used for determining the combined quality metric based on the image quality metrics. Thereby, the determination of the combined quality metric may be adjusted based on user input provided by the radiologist.

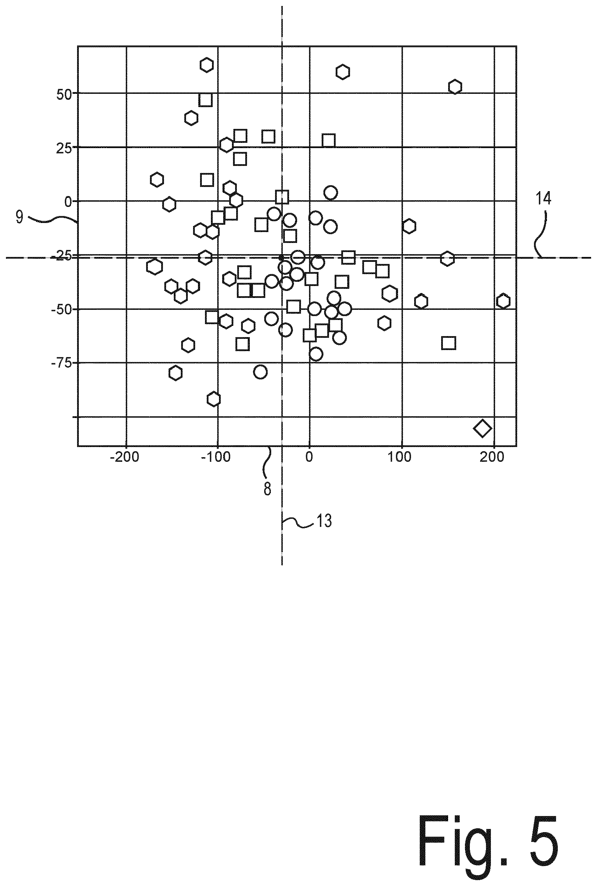

Fig. 5 is a schematic illustration of a graphical representation, which is presented to the radiologist using a graphical user interface of central computer system. In a similar manner as is shown in Fig. 4, each image is illustrated as an icon in a coordinate system having two axes, wherein each of the axes represents one of the image quality metrics. Thereby, for each of hte images, the graphical representation is indicative of a spatial relationship between the respective image and the coordinate system. As is further shown in Fig. 5, each icon is indicative of the combined quality metric of the respective image, since each of the icons has a shape, which is indicative of a level of the combined quality metric: circles represent values of the combined quality metric, which indicate high image quality, squares indicate values of the combined quality metric, which indicate a medium level of image quality and hexagons represent values of the combined quality metric which indicate a low image quality. Thereby, the output is indicative of the combined quality metric.

It is conceivable that for each of the images, the combined quality metric of the respective image is indicated by other means, such icons, which are displayed in different color or which include a number.

It also is conceivable that the coordinate system is a one-dimensional coordinate system of one single image quality metric or that the coordinate system is a three-dimensional spatial coordinate system. It is also conceivable that one or more dimensions are illustrated using a color coding, icons and/or numbers so that it is possible to provide a graphical representation of more than three image quality metrics. The graphical user interface may be configured to receive user input for selecting one or more image quality metrics, which are used to generate the graphical representation. Additionally or alternatively, the central computer system may be configured to automatically or interactively (i.e. requiring user intervention) determine one or more image quality metrics based on a predefined criterion. The predefined criterion may depend on an intended diagnosis.

The graphical user interface of the central computer system is further configured so that the radiologist can select one or more of the images. By way of example, the graphical user interface may be configured so that each of the icons is indicative of a user-selectable region of the graphical user interface. The user-selectable regions may be selectable using a computer mouse of the data processing system. In response to the selection, the central computer system displays the selected image allowing the radiologist to review, whether the determined combined quality metric adequately indicates, whether or not the image is suitable for diagnosis.

Therefore, the data processing system may be configured to receive, via the graphical user interface, a user-specified selection of one or more of the medial images. In response to the user-specified selection, the central computer system displays the selected one or more images to the user and requests the user to input user input, which, for each of the selected one or more medical images, is indicative of a user-specified quality rating for the respective image.

In a same manner, as has been discussed in connection with Fig. 3, the graphical user interface may display a graphical representation, which indicates parameters, which have been used for registering the image with the atlas. Displaying the image using the graphical representation allows the radiologist to more easily check, whether the image is of diagnostic image quality.

Additionally or alternatively, the central computer system may be configured to register the medical image with an atlas using one or more rigid transformations. The central computer system may further be configured to display at least a portion of the transformed image to the user using the graphical user interface.

The rigid transformations may include a rotation transformation, a scaling transformation and/or a translation of the image. Since the atlas has a fixed position, orientation and scale, applying the rigid transformations to a plurality of different images generates a representation of the anatomy, which is coherent between these images. Therefore, using these rigid transformations makes it easier for the radiologist to compare images and thereby to recognize anomalies within the image. This allows for a more time-efficient and more reliable review of the image quality conducted by the radiologist.

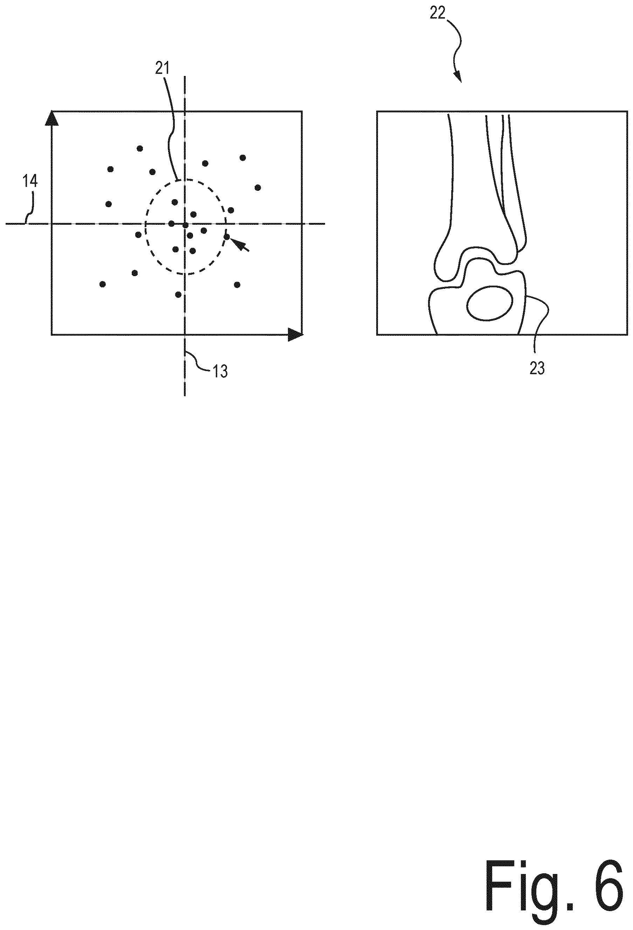

Fig. 6 schematically illustrates in more detail the operation of the graphical user interface of the central computer system 7 (illustrated in Fig. 1). In the illustrated exemplary embodiment, the graphical user interface is configured to display a graphical representation 21, which is indicative of a limit for the combined quality metric. The limit may be indicative of a range of values of the combined quality metric, for which the image if of diagnostic image quality. Therefore, the limit is a graphical representation for a classification of the images into two or more classes (i.e. diagnostic and non-diagnostic).

Therefore, similarly as has been discussed in connection with Fig. 4, the graphical representation 21 in Fig. 6 indicates a limit 21 for the combined quality parameter, which separates images having a diagnostic image quality from images having a non-diagnostic image quality.

As has further been explained in connection with Fig. 4 above, the graphical representation 21 (shown in Fig. 6) of the limit does not have the shape of a rectangle, since the combined quality metric takes into consideration the interaction between the image quality metrics in their effect on the combined quality metric.

The graphical representation 21 allows the radiologist to select those images, which are close to the limit in order to check, whether the limit represents an adequate separation between images of diagnostic quality and images of non-diagnostic quality. The graphical user interface is configured so that the radiologist can select an image using the mouse cursor and the central computer system 7, in response to the selection, displays the image selected image to the user.

As is further schematically illustrated in Fig. 6. Since the graphical user interface allows selection of images which are close to the determined quality threshold 21, it is easier to determine based on a large amount of images, whether the calculated combined quality metric and the determined threshold 21 adequately indicates whether or not images are diagnostic. Specifically, it is easier for the radiologist select those images, for which it is necessary to provide a user-specified quality rating in order to efficiently adapt the algorithm for determining the combined quality metric and/or to efficiently adapt the algorithm for determining the limit 21 for the combined quality parameter.

The central computer system 7 may further be configured to select images based on the limit and further based on the determined combined quality metric. Specifically, the selection may be so that it is not necessary for the radiologist to determine images, which need to be reviewed.

The central computer system 7 is further configured to receive, via the graphical user interface, for one or more of the images, input from the radiologist, which is indicative of a user-specified quality rating for the respective image. In particular, the user-specified quality rating may include an indication of whether or not the image is of diagnostic image quality.

Based on the input received via the user interface, the central computer system adjusts the limit for the combined quality metric (which is an example for algorithm for classifying the images based on the combined quality metric) and/or the central computer system adjusts the algorithm for determining the combined quality metric. It is also possible that the central computer system determines, based on the user input, a new algorithm for classifying the images and/or a new algorithm for determining the combined quality metric.

The adaptation or determination of the criterion for selecting images based on the combined user input and/or the adaptation or determination of the algorithm for determining the combined quality metric may include a machine learning process in which the user's input (i.e. the indication whether or not the image is suitable for diagnosis and/or the user-specified quality rating) is used as training data.

Additionally or alternatively, the determination of the algorithm for classifying the images based on the combined user input and/or the adaptation of the algorithm for determining the combined quality metric may be performed using dimensionality reduction techniques, such as kernel principal component analysis. By way of example, such techniques may be used to simplify the determination of the limit by reducing the required number of images to be reviewed by the radiologist. Specifically, for a given limit, these techniques can be used to find a lower dimensional phase-space representation of the limit for the combined quality metric. The lower dimensional phase-space representation may facilitate a graphical illustration of the limit via the graphical user interface and may also make the adaptation of the limit for the combined quality metric more efficient. After having adapted the limit, the dimensionality reduction technique may be applied again for further simplifying the phase-space.

The determination or adaptation of the algorithm for classifying the images and/or for determining the combined quality metric may be performed using one or a combination of: a maximum likelihood model and a machine learning algorithm. The machine learning algorithm may be configured for supervised and/or unsupervised learning. The machine learning algorithm may be implemented using a support vector machine or an artificial neural network.

Specifically, the maximum likelihood model may be implemented using the assumption that the image quality metrics span an Euclidean space and that the distribution of one of a plurality of predefined classes (such as the class of diagnostic images) in this space is comparatively compact and that a function f(p) is known to approximate it, such as a Gaussian normal distribution.

According to an embodiment, the central computer system is configured to generate a maximum likelihood model that indicates, for a plurality of points p in the Euclidean space, the probability that the corresponding image is a member of one of the plurality of predefined classes. By way of example, the predefined classes include a class of diagnostic images and a class of non-diagnostic images. The central computer system may further be configured to use a threshold Θ for deciding, for a combination of image quality metrics, of which class the corresponding image is a member (e.g. diagnostic if f(p) > Θ or non-diagnostic if f(p)<= Θ). The corresponding boundary may represent a Mahalanobis distance.

A classification algorithm, which uses machine learning may be implemented using a support vector machine and/or an artificial neural network. A description of support vector machines, which can be used for the embodiments described herein, can be found in the book "The nature of statistical learning", written by Vladimir Vapnik in 1995 and published by "Springer Science+Business Media", New York.

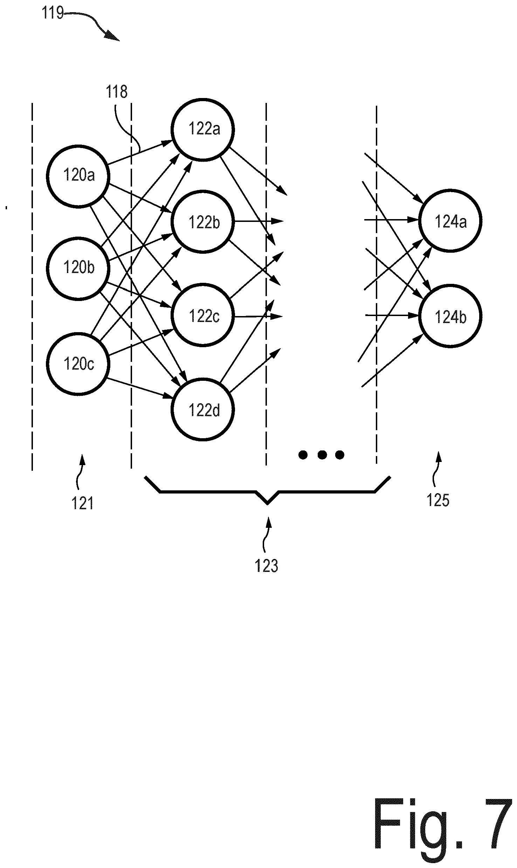

Fig. 7 is a schematic illustration of an artificial neural network (ANN) 119. The ANN may be used by the computer systems 5a, 5b and 5c (shown in Fig. 1) to perform the segmentation of the medical images for determining the combined quality metric. The same configuration of the ANN may be used by the central computer system 7 to determine the algorithm for determining the combined quality metric and/or for determining the algorithm for classifying the images.

As can be seen from Fig. 7, the ANN 19 includes a plurality of neural processing units 120a, 120b, ... 124b. The neural processing units 120a, 120b, ... 124b are connected to form a network via a plurality of connections 118, each of which having a connection weight. Each of the connections 118 connects a neural processing unit of a first layer of the ANN 119 to a neural processing unit of a second layer of the ANN 119, which immediately succeeds or precedes the first layer. As a result of this, the artificial neural network has a layer structure which includes an input layer 121, at least one intermediate layers 123 (also denoted as hidden layer) and an output layer 125. In Fig. 4a, only one of the intermediate layers 123 is schematically illustrated. However, it is contemplated that the ANN 119 may include more than 2 or more than 3, or more than 5 or more than 10 intermediate layers. Specifically, the ANN may be configured as a deep artificial neural network. The number of the layers may be less than 200, or less than 100, or less than 50.

A description of an ANN, which may be used for the embodiments described in the present disclosure, is described in the article "Foveal fully convolutional nets for multi-organ segmentation", written by Tom Brosch and Axel Saalbach and published in SPIE 10574, Medical Imaging 2018: Image Processing, 105740U. The contents of this article is incorporated by reference herein for all purposes.

The ANN may be configured as a convolutional neural network. The term "convolutional neural network" may be defined herein as an artificial neural network having at least one convolutional layer. A convolutional layer may be defined as a layer which applies a convolution to the previous layer. The convolutional layer may include a plurality of neurons, wherein each neuron receives inputs from a predefined section of the previous layer. The predefined section may also be called a local receptive field. The weights for the predefined section may be the same for each neuron in the convolutional layer. Thereby, the convolutional layer may be defined by the two concepts of weight sharing and field accepting. The ANN may include one or more subsampling layers. Each of the subsampling layers may be arranged subsequent (in particular immediately subsequent) to a respective convolutional layer. The subsampling layer may be configured to downsample the output of the preceding convolution layer along the height dimension and along the width dimension. The number of convolution layers, which are succeeded by a pooling layer may be at least 1, at least 2 or at least 3. The number of layers may be less than 100, less than 50, or less than 20.

The above embodiments as described are only illustrative, and not intended to limit the technique approaches of the present invention. Although the present invention is described in details referring to the preferable embodiments, those skilled in the art will understand that the technique approaches of the present invention can be modified or equally displaced without departing from the protective scope of the claims of the present invention. In particular, although the invention has been described based on a projection radiograph, it can be applied to any imaging technique which results in a projection image. In the claims, the word "comprising" does not exclude other elements or steps, and the indefinite article "a" or "an" does not exclude a plurality. Any reference signs in the claims should not be construed as limiting the scope.

## Claims

1. A system (1) for analysis of medical image data, which represent a two-dimensional or three-dimensional medical image , which has been acquired from a human subj ect;
wherein the system comprises a data processing system, which is configured to:
read and/or determine, for the medical image, a plurality of image quality metrics; and to
automatically or interactively determine a combined quality metrics based on the image quality metrics;
wherein the data processing system is configured so that the determination of the combined quality metrics takes into account an interaction between the image quality metrics in their combined effect on the combined quality metrics.

2. The system (1) of claim 1, wherein the interaction between the image quality metrics is so that a change of the combined quality metrics, which is caused by a change of a first one of the quality metrics is compensable by a change of one or more of the remaining quality metrics.

3. The system (1) of claim 1 or 2,
wherein each of the image quality metrics is associated with a respective predefined optimum value or predefined optimum range;
wherein the interaction between the image quality metrics is so that an increased deviation from the optimum range or optimum value of a first one of the metrics is compensable by a decreased deviation from the optimum range or optimum value of one or more of the remaining image quality metrics.

4. The system (1) of any one of the preceding claims, wherein one or more or each of the quality metrics is indicative of one or more parameters of a position and/or one or more parameters of an orientation of an imaged body portion relative to a component of the imaging unit or relative to a further body portion.

5. The system (1) of any one of the preceding claims,
wherein the system (1) is configured to determine, for one or more of the images, based on the determined combined quality metric, a parameter or state variable representing a change of imaging conditions for using the imaging unit;
wherein the changed imaging conditions are configured to change one or more of the image quality metrics.

6. The system (1) of any one of the preceding claims, wherein the determination of the image quality metrics include:
segmenting at least a portion of the image; and/or
registering the image with an atlas using the segmented image.

7. A system (1) for analysis of medical image data, which represent a plurality of medical images, each of which being a two-dimensional or a three-dimensional image;
wherein the medical images are acquired from one or more human subjects using one or more medical imaging units (2a, 2b, 2c), wherein each of the medical imaging units (2a, 2b, 2c) is configured to acquire medical images;
wherein the system (1) comprises a data processing system, which is configured to:
read and/or generate, for each of the medical images, one or more quality metrics; and to
receive, via the user interface, for at least a portion of the images, user input indicative of a user-specified quality rating for the respective image;
wherein the data processing system is further configured to:
-
(a) determine or adapt an algorithm for determining a combined quality metric based on the image quality metrics and based on the user input; and/or to
(b) determine or adapt an algorithm for classifying the images or selecting a portion of the images, wherein the classifying or the selecting is based on the user input and based on the combined quality metric;
- wherein the combined quality metric depends on an interaction between the image quality metrics in their combined effect on the combined quality metrics.

8. The system (1) of claim 7, wherein the system (1) is configured to display, via the user interface:
output, which is indicative of the combined quality metric for at least a portion of the images; and/or
output, which is indicative of a classification of the medical images, which classifies the images based on the combined quality metric.

9. The system of any one of claims 7 or 8, wherein the system is configured to output, via the user interface, a graphical representation which is indicative of a coordinate system of one or more of the image quality metrics;
wherein, for one or more of the images, the graphical representation is further indicative of a spatial relationship between the respective image and the coordinate system.

10. The system of any one of claims 7 to 9, wherein the data processing system is configured to receive, via the user interface, a user-specified selection of one or more of the images for inputting the user input which is indicative of the user-specified rating for the images, which are selected by the user-specified selection.

11. A computer-implemented method for analysis of medical image data, which represent a two-dimensional or three-dimensional medical image, wherein the image is an image of at least a portion of a human subject; wherein the method is performed using a data processing system, wherein the method comprises:
reading and/or determining, using the data processing system, for the medical image, a plurality of image quality metrics; and to
automatically or interactively determining, using the data processing system, a combined quality metrics based on the image quality metrics;
wherein the determining of the combined quality metrics takes into account an interaction between the image quality metrics in their combined effect on the combined quality metrics.

12. A computer-implemented method for analysis of medical image data, which represent a plurality medical images, each of which being a two-dimensional or a three-dimensional image; wherein the medical images are images of portions of one or more human subjects;
wherein the method is performed using a data processing system, wherein the method comprises
reading and/or generating, using the data processing system, for each of the medical images, one or more quality metrics;
receiving, via a user interface of the data processing system, for at least a portion of the images, user input indicative of a user-specified quality rating for the respective image;
wherein the method comprises at least one of the following:
- (a) determining or adapting, using the data processing system, an algorithm for determining a combined quality metric based on the image quality metrics and based on the user input; and/or
- (b) determining or adapting, using the data processing system, an algorithm for classifying the images or selecting a portion of the images, wherein the classifying or the selecting is based on the user input and based on the combined quality metric;
wherein the combined quality metric depends on an interaction between the image quality metrics in their combined effect on the combined quality metrics.

13. A program element for analysis of medical image data, which represent a two-dimensional or three-dimensional medical image, wherein the image is an image of at least a portion of a human subject; wherein the method is performed using a data processing system,
wherein the program element, when being executed by a processor of the data processing system, is adapted to carry out:
reading and/or determining, using the data processing system, for the medical image, a plurality of image quality metrics; and to
automatically or interactively determining, using the data processing system, a combined quality metrics based on the image quality metrics;
wherein the determining of the combined quality metrics takes into account an interaction between the image quality metrics in their combined effect on the combined quality metrics.

14. A program element for analysis of medical image data, which represent a plurality medical images, each of which being a two-dimensional or a three-dimensional image; wherein the medical images are images of portions of one or more human subjects;
wherein the program element, when being executed by a processor of the data processing system, is adapted to carry out:
- reading and/or generating, using the data processing system, for each of the medical images, one or more quality metrics;
- receiving, via a user interface of the data processing system, for at least a portion of the images, user input indicative of a user-specified quality rating for the respective image;
- wherein the program element, when being executed by the processor is further configured to carry out at least one of the following:
- (a) determining or adapting, using the data processing system, an algorithm for determining a combined quality metric based on the image quality metrics and based on the user input; and/or
- (b) determining or adapting, using the data processing system, an algorithm for classifying the images or selecting a portion of the images, wherein the classifying or the selecting is based on the user input and based on the combined quality metric;
wherein the combined quality metric depends on an interaction between the image quality metrics in their combined effect on the combined quality metrics.

15. Computer program product having stored thereon the computer program element of claim 13 and/or claim 14.
